Europäisches Patentamt

European Patent Office

Office européen des brevets

(11) Publication number: **0 238 106**
**A1**

# EUROPEAN PATENT APPLICATION

(21) Application number: 87200098.9

(51) Int. Cl.⁴: **A61M 25/00**

(22) Date of filing: 23.01.87

(30) Priority: 11.02.86 NL 8600338

(43) Date of publication of application:
23.09.87 Bulletin 87/39

(84) Designated Contracting States:
AT BE CH DE ES FR GB GR IT LI LU NL SE

(71) Applicant: **Reytenbagh, Louis Johannes Karel Jozef**
**Hofkampstraat 56**
**NL-7607 NJ Almelo(NL)**

(72) Inventor: **Reytenbagh, Louis Johannes Karel Jozef**
**Hofkampstraat 56**
**NL-7607 NJ Almelo(NL)**

(74) Representative: **van der Beek, George Frans et al**
**Nederlandsch Octrooibureau Johan de Wittlaan 15 P.O. Box 29720**
**NL-2502 LS 's-Gravenhage(NL)**

(54) Catheter provided with positioning means.

(57) Catheter, especially intended for catheterisation of the heart, blood vessels or other vascular and hollow body structures, consisting of a flexible tube construction (6) provided with positioning means which are active at the distal end, and can be operated independently or silmultaneously from the proximal end, therefore outside the body, in order to position the distal tip of the catheter with respect to the wall of the body structure, said positioning means are consisting of at least two, but preferably three or more flexible supprt wires (11), to be stressed with pull and pressure, which are conducted at almost full lenght through coaxial channels (10) and parallel grooves (11) situated around the central part of the tube construction (6) in which the distal end of the support wires (11) is attached to the top of the tube construction (6),and in which the support wires (11) over a certain distance before the top can be bent outward in a curved manner independently of each other, starting from the parallel grooves (12) in a radial direction.

fig-3

## CATHETER PROVIDED WITH POSITIONING MEANS

The invention relates to a catheter especially intended for catheterisation of the heart, blood vessels or other tubular and hollow body structures, consisting of a flexible tube construction with or without a central lumen through which a guide wire can be conducted.

Catheterisation of the heart or vessels of a human being and of most kinds of animals is, in general, accomplished via a peripheral artery or vein, such as the brachial artery, the femoral artery or vein, but also other access routes are feasible.

Various kinds of catheters and comparable devices are used to perform all sorts of measurements, determinations and actions (e.g. administering liquids or drugs) on the human or animal body.

By way of these catheters, blood is obtained for oxygen determinations or the like. Also, pressures can be measured in the various compartments of the heart and the vascular system and contrast media can be administered to visualize the various compartments of the heart and the coronary or other vessels, including bypasses, generally after the catheter has been introduced selectively into the relevant heart compartment or vessel.

Sometimes it is difficult to selectively position the relevant catheter(s) into the intended place; sometimes it presents difficulties to reach every heart compartment desired or every coronary artery or other vessel desired. Therefore, many types of catheters have been designed to reach the relevant compartments of the heart and the vascular system as easily as possible, also when the morphology is complicated by pathological processes or anatomical variations. Amongst other things steerable guide wires, pre-shaped catheters and such are used.

A problem in this area is that it is often difficult to exactly position such a catheter in, for instance, the aorta. In adults, the aorta has a diameter of some 40 mm and the catheter often moves in a direction other than intended (especially often towards the wall of the vessel).

The above-mentioned problem especially occurs if one intends to pass a so-called stenotic - (narrowed) aortic valve in order to reach the left ventricle of the heart, or if the ascending part of the aorta (aorta ascendens) is very large, as can be the case with aortic valve stenosis, aortic valve insufficiency or other pathological processes of the aorta. It should be considered that, especially in a number of these situations, it is very important to selectively reach the left ventricle in order to determine the anatomical and functional characteristics of this ventricle, and to measure the pressure gradient between the left ventricle and the aorta, which has far-reaching consequences for the policy to be pursued and the treatment of choice.

For instance in the case of an aortic valve stenosis, the opening of the aortic valve is often restricted, the maximum diameter of the valve opening (ostium) being severely reduced, and so passing the narrowed valve is often very difficult.

The catheter time and again glances off, as it were, against the narrowed, often calcified valve. Valve stenosis and deposition of calcified substances often simultaneously occur, the commissurae of the valve leaflets have often grown out of shape.

Moreover, the risk exists that pieces of calcium, trombi (clots) and other substances of the pathological valve are pushed off by the catheter whereupon an embolism may occur with severe consequences for the patient (cerebro-vascular accident, partial paralysis, cholesterol embolisms, peripheral vascular obstruction and so on). This risk in creases as the attempts to pass the valve are protracted, which is often the case with the current techniques.

Also in cases where the valve is not narrowed but the aortic root is large, passing the valve with the presently known catheters is difficult, because the catheter tends to slip away towards the wall of the vessel (aorta).

The invention intends to supply a catheter with the characteristic that the distal tip of the catheter can be conducted to the right place, respectively manoeuvred into the right position so that, for instance in case of a heart catheterisation, for which a catheter with a central lumen is used, a guide wire can be inserted through the opening of the aortic valve more easily and involving less risk.

According to the invention this purpose is reached in that the catheter is provided with positioning means which are active at the distal end and can be operated independently or simultaneously from the proximal end, therefore from outside the body, allowing the positioning of the distal tip of the catheter or the distal tip of the catheter or the distal mouth of the central lumen with regard to the wall of the hollow body structure.

The principle can also be applied to stablely position a catheter in a vascular structure in situations in which the fixed position of the catheter in the right place is essential, for example in measuring blood flow velocity. In that case, for example, registration equipment can be fixed on the tip of the catheter. The central lumen can be omitted if desirable. The above mentioned indications for use of this catheter are just examples. It is to be understood, however, that these are given by way of illustration and not as a limitation and that other applications are possible and within the scope of the invention.

Preferably the positioning means consist of at least two, but preferably three or more flexible support wires to be stressed with pull and pressure, which are, almost at full length, conducted through parallel channels and aligned grooves situated around the central part of the tube construction which may contain a central lumen, whereby the distal ends of the support wires are attached to the top of the tube construction and the support wires can be bent in or outward in a curved manner independently of each other in a radial direction, over a certain distance before the top.

By these relatively simple measures it becomes possible to position the distal end of the catheter in accordance with the invention into the right desired place.

The invention will be further clarified for an embodiment with a central lumen by means of attached drawings, in which:

Figure 1 shows the heart and the aorta with there in the catheter according to the invention in a positioning stance;

Figure 2 shows the catheter during the insertion;

Figure 3 shows the catheter in the positioning stance in accordance with figure 1;

Figure 4 is a cross-section along the line IV-IV of figure 2 at a larger scale; and

Figure 5 is a cross-section along the line V-V of figure 2 at a larger scale.

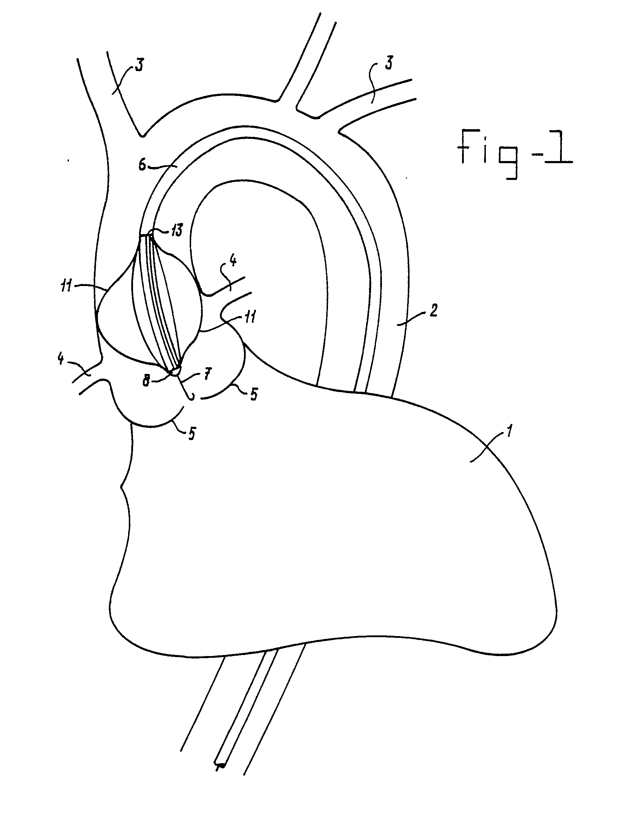

In figure 1, the left ventricle of a human heart is indicated by 1, the aorta by 2, the branches to neck, head and arms by 3, the coronary arteries by 4 and the aortic valve consisting of three leaflets by 5 (only two leaflets are visible in figure 1).

In order to enter the left ventricle (1) with the distal end of a catheter, the opening of the aortic valve (5) has to be passed.

A conventional catheter consists of a flexible tube construction with one central lumen, through which a flexible guide wire can be conducted. Most of the known catheters are also advanced by means of a guide wire inserted in this central lumen.

Also the catheter in accordance with this invention consists of a flexible tube construction (6) with or without a central lumen. The shown embodiment is provided with a central lumen (9). In that case a guide wire (7) can be used. This guide wire can, for instance, have a straight or J-shaped end.

In the case of a conventional catheter without positioning devices, the distal end (8) of the catheter or the protruding end of the guide wire (7) must be passed through the central opening of the aortic valve at exactly the right moment. As the catheter tends to move along the wall of the aortic root, it is sometimes difficult to find the opening of the valve (5). Sometimes it requires protracted manoeuvring along them, especially for instance with aortic valve stenosis (narrowing).

Passing the aortic valve opening is usually not problematic with a normal aortic valve, but may be very difficult with a pathological aortic valve which, amongst other things, hardly opens, due for example to deformation or fusing of the three leaflets. Moreover, the leaflets may be covered with calcium, cholesterol, cell debris and such, because of which, in manipulating the catheter, the risk exists that particles are pushed loose (embolized) and enter the bloodstream, with fatal consequences for the patient.

Therefore it is desirable that, without a lot of manipulating, and without pushing particles loose, the aorta valve opening can be found and the end of the guide wire can be passed through successfully.

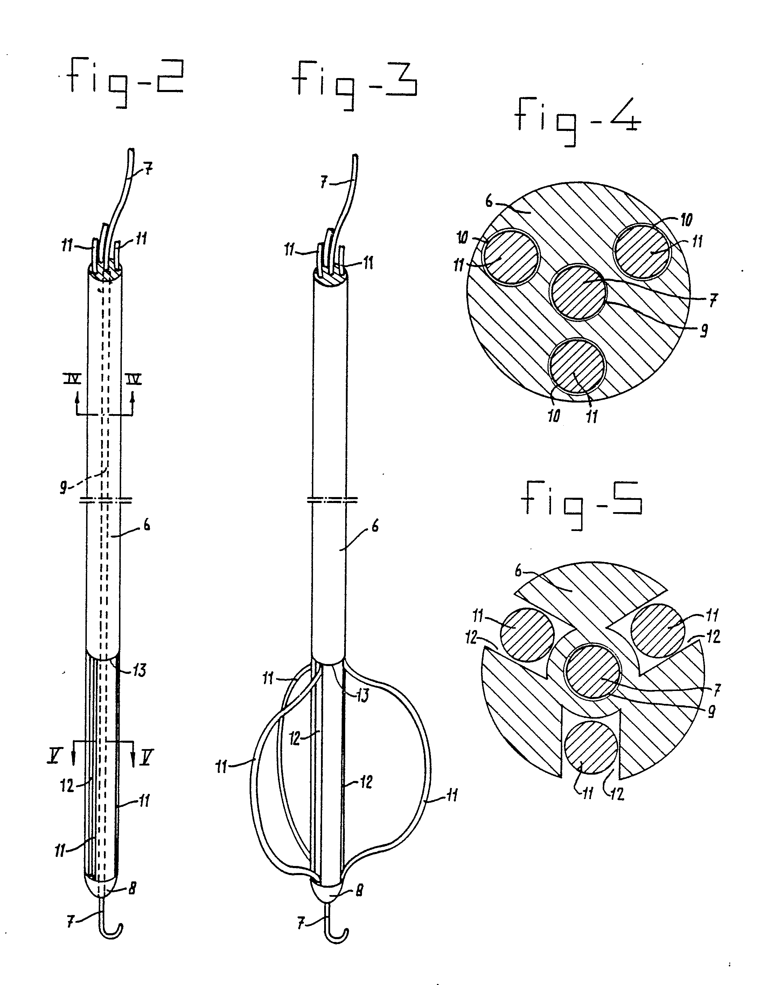

To make this possible, the tube construction of the catheter in accordance with the invention is not only provided with a central lumen (9), but with a number of -at least two, but preferably three or more -coaxially running channels (10), see figure 4. Through these channels (10) support wires (11) are extending, which may be stressed with pull as well as pressure.

The tube construction (6) preferably consists of synthetic material, whether or not reinforced. The support wires (11) consist of metal or some other kind of suitable material, not causing damage to the wall of the vessel in which the catheter is positioned.

As appears from figures 2, 3 and 5, over a certain length before the -preferably half-globular - top (8) of the catheter, the tube construction (6) is provided with parallel grooves (12), in which the support wires (11) are extended. The parallel grooves (12) are aligned with the channels (10).

The ends of the support wires (11) are attached to the top of the catheter (8).

The catheter in accordance with the invention must be strong enough to avoid any tearing of the tube construction (6), especially in the point indicated by 13 (see figures 2 and 3).

The support wires (11) as well as the tube construction (6) of the catheter and the central guide wire (7), protrude with their proximal ends from the patient's body.

By moving the support wires (11) independently or simultaneously in and out with regard to the tube construction (6), parts of the support wires (11) will protrude in a curved manner from the parallel grooves (12), or will retract. See figures 1 and 3.

In case of a centric aortic valve opening, all support wires (11) will be bent outwards to the same extent, to adjoin the wall of the aorta root.

In case of a more excentric aortic valve opening, one or two of the support threads will need further bending out than the other(s).

Instead of three support wires, two, four or even more may be used (not shown).

As guide wire (7), a so-called "long guide wire" is used, which is available on the market. "Long guide wire" means a guide wire which is at least twice as long as the tube construction of the catheter, in order to make changing the catheter via the guide wire possible.

When, by means of the catheter in accordance with the invention, the end of the guide wire (7) has reached the left ventricle (1), the support wires - (11) are straightened: see figure 2.

The catheter in accordance with the invention can be removed via the long guide wire (7) which remains with its tip in the left ventricle. Subsequently, a suitable conventional catheter can be advanced into the left ventricle via the long guide wire (7) that was left behind. This may, for instance, be a conventional catheter of which the distal end has the shape of a pigtail (pigtail catheter), but also other catheters, for example a balloon valvuloplasty catheter to dilate a stenotic aortic valve, are possible.

When the distal end of the conventional catheter has reached the left ventricle, the guide wire - (7) can be removed.

With this conventional catheter it is possible to measure the pressure in the left ventricle, to take blood samples, to administer contrast media in order to depict the left ventricle, and so on.

If required, the catheter in accordance with the invention could also be inserted into the left ventricle after the central guide wire (7) has been inserted into this ventricle. After removal of the guide wire, several functions such as measuring pressure, administering drugs and taking blood samples with the catheter in accordance with the invention may be performed. As this catheter is less suitable for depicting the left ventricle, for this purpose the catheter in accordance with the invention is changed via the long guide wire (7), for instance for a known pigtail catheter.

Thus it is possible to pass a stenotic aortic valve in a simple way even if this was difficult or impossible until now. Moreover, with the catheter in accordance with the invention the number of abortive attempts to pass the valve will diminish, thus reducing the above-outlined risks of embolic complications.

This also accounts for situations in which the aorta ascendens is enlarged.

Variations to the concept described above might also be applied to other invasive operations upon the human body, in which positioning is relevant. For example, registration equipment can be fixed to the tip of the catheter to measure blood flow velocity in that particular spatial position in an artery where velocity is highest. For this application the central lumen of the catheter may be omitted if desired. Other applications where positioning is important are within the scope of this invention.

The catheter in accordance with the invention is straight, flexible and supple (compliant), and disposes of an optional central axial lumen and a number of coaxial channels. The coaxial channels contain the positioning means (support wires) and end in the longitudinal grooves on the distal end of the catheter. The central lumen, if present, serves:

1. to facilitate introduction and advancement of the catheter itself, if necessary, by way of a guide wire via a peripheral vessel, see fig 1.

2. To insert a suitable guide wire which has to pass the opening of for example the aortic valve, after the catheter has been conducted towards the aortic valve, (the guide wire sub. 1 and 2, in general, will be the same). Usually, pushing the catheter from a peripheral artery (for example brachial artery or femoral artery) to the heart is accomplished successfully, with the guide wire somewhat protruding from the catheter and the distal end of the guide wire being supple.

3. To take blood samples, measure pressures, administer drugs or other agents, and so on, if desired.

The guide wires do not differ essentially from existing types and may be straight or J-shaped, whereas the point may be made stiff or supple - (compliant).

By means of the catheter described above, a number of aspects of heart catheterisation and other invasive manipulations become more easily practicable. With the shown embodiment it becomes easier and with less risk for the patient involved, to pass the aortic valve in situations where this used to be difficult or impossible, and involved the risk of embolic complications. It is also possible to reliably measure blood flow velocity in a desired position without shifting the catheter. Many other applications in which positioning is relevant are possible.

The same principle can also be applied in catheterisation of other vascular and hollow body structures in the human (or animal) body, where positioning is important. The principle in general allows posi tioning a catheter in a stable way in any desired spatial position in a blood vessel or hollow body structure.

The diameter of the catheter in accordance with the invention doesn't need to differ essentially from the diameter of the conventional catheters. An appropriate measure for cardiovascular applications is, for instance, 7-14 French (this is a diameter of 2,2 -4,5 mm approximately). With such a diameter it is technically possibly to apply one central lumen and several coaxial channels and grooves. If necessary and technically possible a smaller sized catheter, e.g. 5 or 6 French, can be constructed for use in smaller arteries.

## Claims

1. Catheter, especially intended for catheterisation of the heart, blood vessels or other vascular and hollow body structures, consisting of a flexible tube construction with or without a central lumen, through which a guide wire can be conducted, characterized in that the catheter is provided with positioning means which are active at the distal end, and can be operated independently or simultaneously from the proximal end, therefore outside the body, in order to position the distal tip of the catheter or the distal mouth of the central lumen with respect to the wall of the body structure.

2. Catheter in accordance with claim 1, characterized in that the positioning means consist of at least two, but preferably three or more flexible support wires, to be stressed with pull and pressure, which are connected at almost full length through the coaxial channels and parallel grooves situated around the central part (with or without lumen) of the tube construction in which the distal end of the support wires is attached to the top of the tube construction, and in which the support wires over a certain distance before the top can be bent outward in a curved manner independently of each other, starting from the parallel grooves in a radial direction.

3. Catheter in accordance with claim 2, characterized in that the tube construction is strong enough, especially at the transition from the coaxial channels to the parallel grooves, to avoid any tearing of the tube construction.

fig-1

# fig-2

# fig-3

# fig-4

# fig-5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US-A-3 704 711  (S.C. PARK)<br>* Column 3, lines 38-43;  figures * | 1,2 | A 61 M   25/00 |
| A | US-A-3 568 659  (J.N. KARNEGIS)<br>* Claim 1; figure 2 * | 2 | |
| A | US-A-4 154 242  (Z.A. TERMANINI)<br>* Abstract; figure 1 * | 2 | |

TECHNICAL FIELDS
SEARCHED (Int. Cl.4)

A 61 M
A 61 B
A 61 N

The present search report has been drawn up for all claims

| Place of search | Date of completion of the search | Examiner |
|---|---|---|
| THE HAGUE | 10-04-1987 | VANRUNXT J.M.A. |